(19) Europäisches Patentamt
European Patent Office
Office européen des brevets

(11) **EP 2 687 261 A1**

(12) **EUROPEAN PATENT APPLICATION**

(43) Date of publication:
**22.01.2014 Bulletin 2014/04**

(51) Int Cl.:
**A61N 5/10** $^{(2006.01)}$ **G06T 11/00** $^{(2006.01)}$

(21) Application number: **13020052.0**

(22) Date of filing: **18.07.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **20.07.2012 JP 2012161796**

(71) Applicant: **SUMITOMO HEAVY INDUSTRIES, LTD. Tokyo 141-6025 (JP)**

(72) Inventor: **Yamaguchi, Takashi Yokosuka-shi, Kanagawa, 237-8555 (JP)**

(74) Representative: **Wagner, Karl H.**
**Wagner & Geyer**
**Gewürzmühlstrasse 5**
**80538 Munich (DE)**

(54) **CT image apparatus for charged particle beam therapy**

(57) A CT image creation apparatus for charged particle beam therapy includes: an image acquisition unit that acquires X-ray image data, which is imaged every predetermined set rotation angle, while rotating a rotating gantry to which an X-ray tube that emits X-rays and an X-ray detector that detects X-rays emitted from the X-ray tube are fixed; a reconstruction unit that reconstructs a CT image on the basis of the X-ray image data; a first detection unit that detects a difference between an angle of the rotating gantry, at which the X-ray image data has been imaged, and the predetermined rotation angle on the basis of the CT image; and a first correction unit that corrects the X-ray image data on the basis of a detection result of the first detection unit. The reconstruction unit reconstructs the CT image on the basis of X-ray image data corrected by the first correction unit.

**Fig.6**

**Description**

Incorporation by Reference

Priority is claimed to Japanese Patent Application No. 2012-161796, filed July 20, 2012, the entire content of each of which is incorporated herein by reference.

BACKGROUND

Technical Field

The present invention relates to a CT image creation apparatus for charged particle beam therapy.

Description of the Related Art

For example, a proton beam irradiation apparatus (charged particle beam therapy apparatus) that irradiates an object with protons accelerated so as to have high energy is used for the treatment of regional disease, such as cancer. Specifically, the treatment is performed by emitting a proton beam from the proton beam irradiation apparatus to damage the cells in the affected part.

Typically, prior to proton beam irradiation, the position of the affected part is specified using an X-ray CT apparatus. In the treatment using a proton beam irradiation apparatus, it is necessary to irradiate the affected part of the body intensively with a proton beam. For this reason, it is important to accurately irradiate the affected part with a proton beam. When determining how to irradiate a proton beam, the result of imaging by the X-ray CT apparatus rather than the proton beam irradiation apparatus is used. Accordingly, it is necessary to perform alignment so that the apparatus error between the proton beam irradiation apparatus and the X-ray CT apparatus is as small as possible. As this method, for example, a configuration to perform data conversion using data for alignment correction for converting the data based on the coordinate system of the X-ray CT apparatus into the data based on the coordinate system of the radiation therapy apparatus is disclosed in the related art.

SUMMARY

According to an embodiment of the present invention, there is provided a CT image creation apparatus for charged particle beam therapy including: an image acquisition unit that acquires X-ray image data, which is imaged every predetermined set rotation angle, while rotating a rotating gantry to which an X-ray tube that emits X-rays and an X-ray detector that detects X-rays emitted from the X-ray tube are fixed; a reconstruction unit that reconstructs a CT image on the basis of the X-ray image data; a first detection unit that detects a difference between an angle of the rotating gantry, at which the X-ray image data has been imaged, and the predetermined rotation angle on the basis of the CT image; and a first correction unit that corrects the X-ray image data on the basis of a detection result of the first detection unit. The reconstruction unit reconstructs the CT image on the basis of X-ray image data corrected by the first correction unit.

BRIEF DESCRIPTION OF THE DRAWINGS

Fig. 1 is a diagram showing the schematic configuration of a proton therapy system including a CT image creation apparatus for charged particle beam therapy according to an embodiment of the present invention.
Fig. 2 is a schematic perspective view of a rotating gantry of the proton therapy system.
Fig. 3 is a side view of the rotating gantry of the proton therapy system.
Fig. 4 is a transverse sectional view showing the cross section along the shaft center of the rotating gantry of the proton therapy system.
Fig. 5 is a perspective view illustrating the configuration of a rotating unit of the rotating gantry.
Fig. 6 is a block diagram illustrating the configuration of the CT image creation apparatus for charged particle beam therapy according to the present embodiment.
Figs. 7A and 7B are diagrams illustrating the positional deviation of the apparatus when creating a CT image.
Fig. 8 is an example of a CT image captured including the positional deviation of the apparatus.
Fig. 9 is an example of a CT image captured including the positional deviation of the apparatus.
Fig. 10 is a flow chart illustrating a method of correcting a CT image.

Fig. 11 is a diagram illustrating the detection of positional deviation.
Figs. 12A and 12B are diagrams illustrating the detection of positional deviation.
Fig. 13 is a diagram illustrating the detection of positional deviation.
Fig. 14 is a diagram illustrating the correction of positional deviation.
Fig. 15 is a diagram illustrating the correction of positional deviation.
Fig. 16 is a CT image reconstructed after correcting the X-ray image data that forms the CT images shown in Figs. 8 and 9.

DETAILED DESCRIPTION

Since the method disclosed in the related art is for alignment between the radiation therapy apparatus and the X-ray CT apparatus, there has been a problem in that the sufficient correction result is not obtained if the method disclosed in the related art is applied to a charged particle beam therapy apparatus that is a larger apparatus.

It is desirable to provide a CT image creation apparatus for charged particle beam therapy capable of creating a CT image for charged particle beam therapy more accurately.

According to the CT image creation apparatus for charged particle beam therapy, the deviation of the angle of the rotating gantry when capturing an X-ray image from the set angle is detected on the basis of a CT image, and the CT image is reconstructed by correcting the X-ray image data on the basis of the detected deviation. Accordingly, since the distortion of a CT image due to the deviation of the angle of the rotating gantry can be modified, it is possible to create a more accurate CT image.

In addition, a second detection unit that detects a difference between a set fixing position of the X-ray tube and a fixing position of the X-ray tube, at which the X-ray image data has been imaged, on the basis of the CT image and a second correction unit that corrects the X-ray image data on the basis of a detection result of the second detection unit may be further provided. The reconstruction unit may reconstruct the CT image on the basis of X-ray image data corrected by the second correction unit.

As described above, by adopting the configuration in which the deviation of the fixing position of the X-ray tube from the set position is detected on the basis of a CT image captured using the rotating gantry and the X-ray image data is corrected on the basis of the detected deviation to reconstruct the CT image, it is possible to create a more accurate CT image.

In addition, a third detection unit that detects a difference between a set fixing position of the X-ray detector and a fixing position of the X-ray detector in the rotating gantry, at which the X-ray image data has been imaged, on the basis of the CT image and a third correction unit that corrects the X-ray image data on the basis of a detection result of the third detection unit may be further provided. The reconstruction unit may reconstruct the CT image on the basis of X-ray image data corrected by the third correction unit.

As described above, by adopting the configuration in which the deviation of the fixing position of the X-ray detector from the set position is detected on the basis of a CT image captured using the rotating gantry and the X-ray image data is corrected on the basis of the detected deviation to reconstruct the CT image, it is possible to create a more accurate CT image.

In addition, the second detection unit may detect deviation of the fixing position of the X-ray tube in the rotating gantry, at which the X-ray image data has been imaged, on the basis of the X-ray image data corrected by the first correction unit.

In the reconstructed CT image, image distortion due to the deviation of the angle of the rotating gantry, among various kinds of positional deviation of the apparatus, is largest. Therefore, the accuracy of correction of a CT image is further improved by performing a modification based on the deviation of the fixing position of the X-ray tube after performing correction related to the deviation of the angle of the rotating gantry.

In addition, a third detection unit that detects deviation of a fixing position of the X-ray detector in the rotating gantry, at which the X-ray image data has been imaged, on the basis of the X-ray image data corrected by the second correction unit and a third correction unit that corrects the X-ray image data on the basis of a detection result of the third detection unit may be further provided. The reconstruction unit may reconstruct the CT image on the basis of X-ray image data corrected by the third correction unit.

As described above, by performing a modification based on the deviation of the fixing position of the X-ray detector after performing correction related to the deviation of the angle of the rotating gantry, the accuracy of correction of a CT image is further improved.

Hereinafter, a CT image creation apparatus for charged particle beam therapy according to a preferred embodiment of the present invention will be described with reference to the accompanying drawings. In the present embodiment, a proton therapy system 1 that is a kind of charged particle beam therapy system including a CT image creation apparatus for charged particle beam therapy will be described. The proton therapy system 1 is an apparatus that emits a proton beam to the lesion (for example, a tumor) inside a patient (object to be examined), for example. In addition, as charged particle beams, not only the proton beam described in the present embodiment but also a heavy particle (heavy ion) beam, an ion meson beam, and the like may be mentioned. The CT image creation apparatus for charged particle beam therapy according to the present embodiment can also be applied to therapies using these charged particle beams.

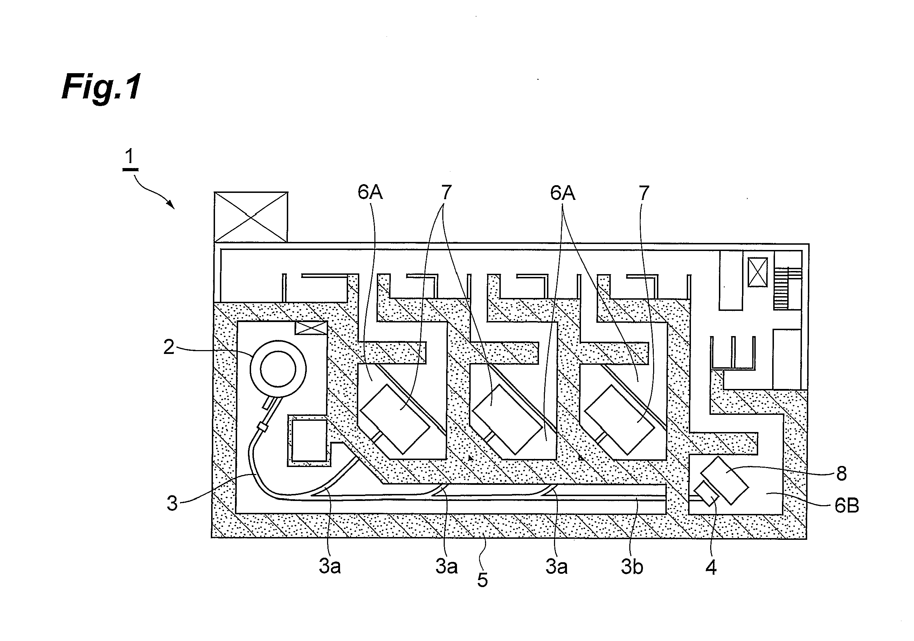

As shown in Fig. 1, the proton therapy system 1 includes a cyclotron (particle accelerator) 2 that accelerates ions generated by an ion source (not shown) and emits a proton beam and a beam transport line 3 to transport the proton beam emitted from the cyclotron 2. In addition, the particle accelerator is not limited to the above-described cyclotron, and a synchrotron, a synchro-cyclotron, a linac (linear accelerator), and the like may also be used.

The proton beam accelerated by the cyclotron 2 is deflected along the beam transport line 3 and is supplied to a fixed irradiation type proton beam irradiation unit 4 and a proton beam irradiation unit of a rotating gantry 7. A deflection magnet for deflecting a proton beam, quadrupole magnets for performing beamforming, and the like are provided in the beam transport line 3. Electromagnets for adjusting the charged particle beam include quadrupole magnets for performing beamforming, a  deflection magnet for deflecting a beam, and the like.

The proton therapy system 1 is housed in a building 5. A plurality of irradiation chambers (laboratories) 6A and 6B formed by radiation shielding walls to prevent the transmission of a radiation are provided in the building 5. The rotating gantry 7 that rotates an X-ray imaging unit, which emits X-rays, and a proton beam irradiation unit integrally is provided in the irradiation chamber 6A. An X-ray imaging apparatus 8 and the proton beam irradiation unit 4 are separately provided in the irradiation chamber 6B.

The building 5 of the present embodiment includes three irradiation chambers 6A and one irradiation chamber 6B, for example. The beam transport line 3 is branched at a predetermined position, and has a line 3a introduced into the irradiation chamber 6A and a line 3b introduced into the irradiation chamber 6B. In the following embodiment, the rotating gantry 7 provided in the irradiation chamber 6A and the proton beam irradiation unit and the X-ray imaging unit included in the rotating gantry 7 will be described.

(Rotation gantry)

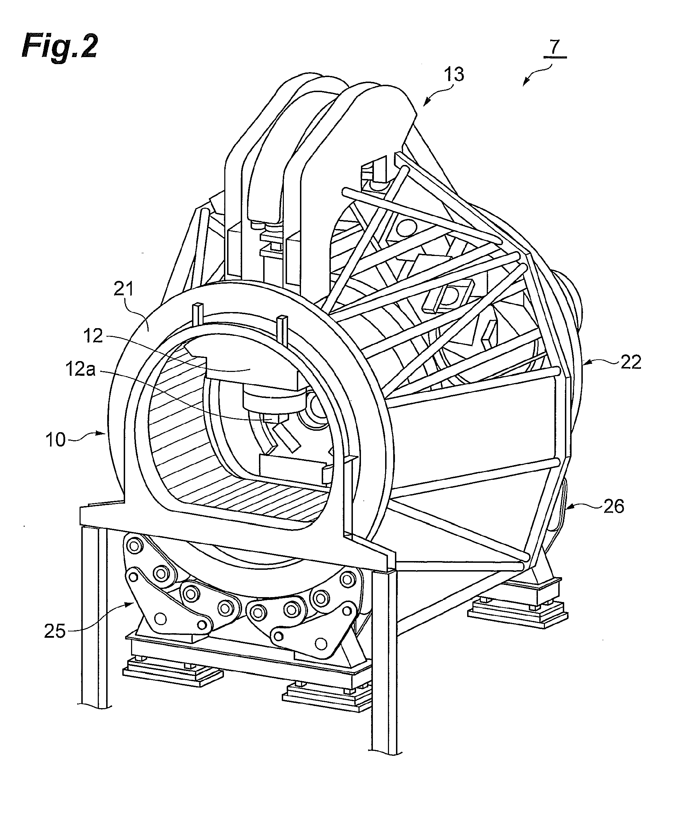

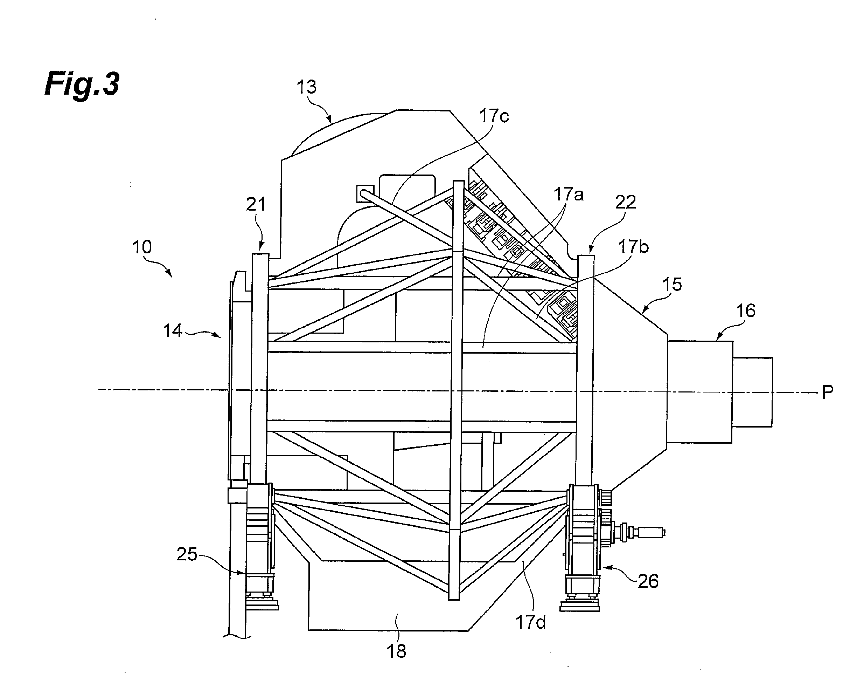

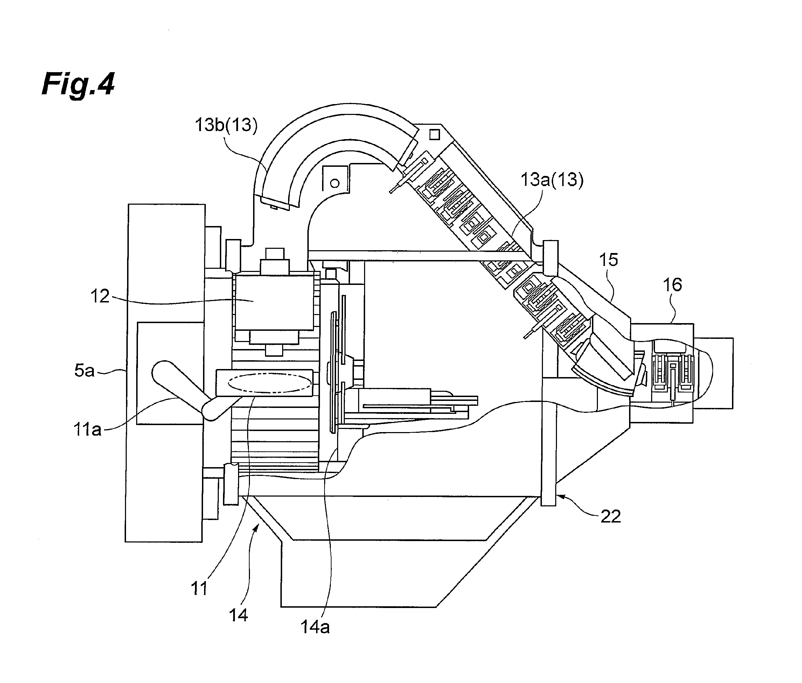

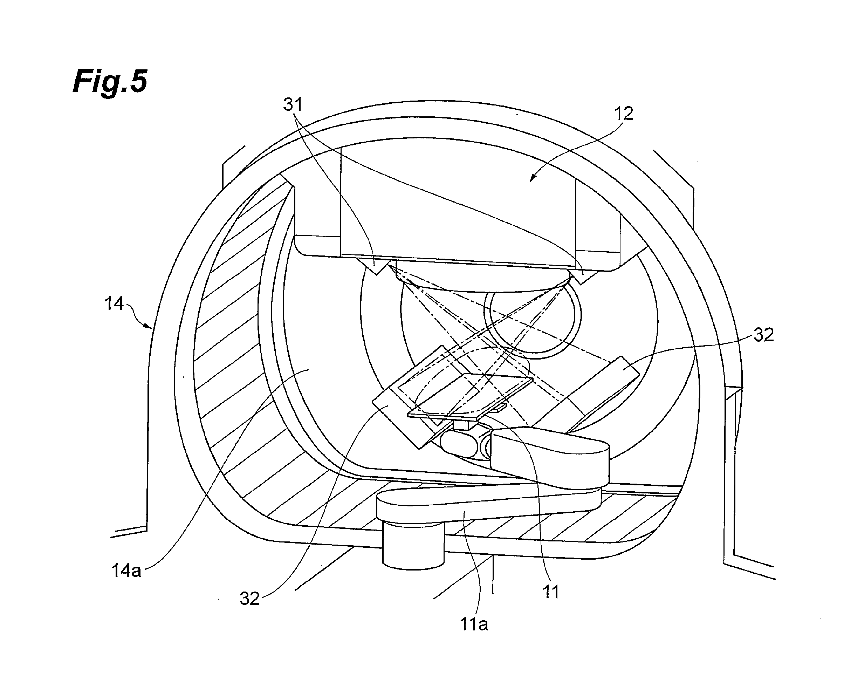

Fig. 2 is a schematic perspective view illustrating the structure of the rotating gantry. In addition, Fig. 3 is a side view of the rotating gantry. Fig. 4 is a partially broken top view of the rotating gantry. Fig. 5 is a schematic perspective view illustrating the vicinity of the treatment chamber of the rotating gantry. In addition, Fig. 4 shows the configuration when the rotating gantry shown in Figs. 2 and 3 are rotated by 90°.

As shown in Figs. 2 to 5, the rotating gantry 7 includes a treatment table 11 (refer to Fig. 5) on which a patient lies, a rotating unit 10 provided so as to surround the treatment table 11, an irradiation unit 12 that is disposed inside the rotating unit 10 and emits a proton beam toward the patient on the treatment  table 11, and an introduction line 13 to introduce the proton beam induced along an induction line 5 to the irradiation unit 12. The rotating gantry 7 is rotated by a motor (not shown), and the rotation is stopped by a braking device (not shown). In addition, in the following explanation, the front of the rotating gantry 7 means a side surface on which the treatment table 11 is placed and the rotating unit 10 is opened so that a patient can be freely moved in and out, and the rear means a side surface on the back side.

The rotating unit 10 can freely rotate, and includes a first cylindrical section 14 and a cone section 15 in order from the front side. The first cylindrical section 14 and the cone section 15 are disposed coaxially and are fixed to each other. In addition, a second cylindrical section 16 provided on the rear side from the rotating unit 10 is disposed coaxially with the cone section 15, but is rotatable relative to the cone section 15.

The treatment table 11 is supported by a support device 11a for which 6-axis control is possible, and is disposed inside the first cylindrical section 14 at the time of treatment. Since the support device 11a is fixed to a floor section 5a of the building 5 instead of the rotating unit 10, it is possible to move the treatment table 11 regardless of the rotation of the first cylindrical section 14.

The irradiation unit 12 is disposed on the inner surface of the first cylindrical section 14, and is directed in a direction of the shaft center of the first cylindrical section 14. The treatment table 11 is disposed near the shaft center (axis of rotation) P inside the first cylindrical section 14. The second cylindrical section 16 has a smaller diameter than the first cylindrical section 14. In addition, the cone section 15 is formed such that the diameter on its one end side is the same as the diameter of the first cylindrical section 14 and the other end has a conical shape so as to be connected to the second  cylindrical section 16. In addition, rear end sides of the cone section 15 and the first cylindrical section 14 are connected to each other by a plurality of struts 17a, which are provided along the outer periphery of the first cylindrical section 14 and extend in the axial direction, and a plurality of struts 17b, which extend in a direction crossing the axial direction.

A ring section 21 that protrudes outward is provided in a front-end outer peripheral portion of the first cylindrical section 14. In addition, a ring section 22 that protrudes outward is also provided in a front-end outer peripheral portion of the cone section 15. The first cylindrical section 14 and the cone section 15 are rotatably supported by a roller device 25 (refer to Figs. 2 and 3) disposed below the ring section 21 of the first cylindrical section 14 and a roller device 26 (refer to Figs. 2 and 3) disposed below the ring section 22 of the cone section 15. Since the outer peripheral portion of the ring section 21 is in contact with the roller device 25 and the outer peripheral portion of the ring section 22 is in contact with the roller device 26, rotational force is applied to the first cylindrical section 14 and the cone section 15 by the roller devices 25 and 26.

The introduction line 13 is connected to the line 3a branched from the beam transport line 3 on the rear side of the rotating gantry 7. The introduction line 13 includes a set of 45° deflection electromagnets and a set of 135° deflection electromagnets. The introduction line 13 has an axial direction introduction line 13a, which is connected to the line 3a (refer to Fig. 1) and extends radially outward along the shaft center P, and a radial introduction line 13b, which is connected to the rear end of the axial direction introduction line 13a and extends radially. In addition, in the introduction line 13, a beam transport pipe (not shown), which is a vacuum pipe through which a proton beam is transported, is provided.

The axial direction introduction line 13a is a path portion that extends from its start end, which is connected to the line 3a on the shaft center P in the second cylindrical section 16, radially so as to be curved by 45° with respect to the shaft center P in the cone section 15 and that has a termination end protruding to the outside of the first cylindrical section 14. In addition, the radial introduction line 13b is a path portion that is curved by 135° from its start end, which is connected to the termination end of the axial direction introduction line 13a, inward in the radial direction of the rotating unit 10 and that has a termination end connected to the irradiation unit 12.

The introduction line 13 is fixed through a strut 17c or the like so as to protrude from the struts 17a and 17b, which connect the first cylindrical section 14 and the cone section 15 to each other, outward in the radial direction of the first cylindrical section 14.

In addition, a counter weight 18 is disposed so as to face the introduction line 13 with the shaft center P interposed therebetween. The counter weight 18 is installed using a strut 17d or the like so as to protrude from the struts 17a and 17b, which connect the first cylindrical section 14 and the cone section 15 to each other, outward in the radial direction of the first cylindrical section 14. By installing the counter weight 18, the weight balance with the introduction line 13 is secured.

In the rotating gantry 7, the first cylindrical section 14, the cone section 15, the introduction line 13, and the counter weight 18 connected to each other by the struts 17a to 17d are integrally rotated around the shaft center P by driving of the roller devices 41 and 42. By rotating the first cylindrical section 14, the cone section 15, the introduction line 13, and the counter weight 18 to change the position of the irradiation unit 12 with respect to the treatment table 11 and then emitting a proton beam from the irradiation unit 12 to the patient on the treatment table 11, a proton beam (charged particle beam) is emitted to the lesion (for example, a tumor) inside the patient (object to be examined) from any direction.

Here, the inside of the first cylindrical section 14 to which the treatment table 11 is fixed will be described with reference to Fig. 5. An emission port 12a of a proton beam that is emitted from the cyclotron 2 and is transported through the introduction line 13 is provided in the irradiation unit 12 provided inside the first cylindrical section 14. In addition, two X-ray tubes 31 to emit X-rays for creating a CT image for charged particle beam therapy are provided outside the irradiation unit 12. These X-ray tubes 31 are located on the plane that is perpendicular to the shaft center P and includes the emission port 12a. The emission port 12a is provided at a position, which is the midpoint of the two X-ray tubes 31, and a conical X-ray beam (cone beam) is emitted from the emission port 12a. In addition, FPDs (Flat Panel Detectors) 32, which are X-ray detectors in which pixels are arranged in a two-dimensional manner in order to receive X-rays emitted from the X-ray tubes 31 and transmitted through an object to be imaged around the shaft center P, are provided at positions facing the X-ray tubes 31 with the shaft center P interposed therebetween.

The FPD 32 is fixed to a wall surface 14a, which is perpendicular to the shaft center P, on the rear side inside the first cylindrical section 14, and rotates according to the rotation of the first cylindrical section 14. In addition, the FPD 32 can be housed at the more rear side than the wall surface 14a. Since the X-ray tube 31 and the FPD 32 are used in order to capture a CT image for charged particle beam therapy, the X-ray tube 31 and the FPD 32 are housed against the irradiation unit 12 and the wall surface 14a upon treatment using a proton beam. In addition, the FPD 32 is pulled out from the wall surface 14a to the front side at the time of use, and is moved to the  position at the time of use. In addition, instead of the FPD 32, for example, an X-ray II (Image Intensifier) capable of receiving X-rays similar to the FPD may also be used.

(Method for creating a CT image for charged particle beam therapy)

Next, a CT image creation apparatus for charged particle beam therapy in the proton therapy system 1 according to the present embodiment will be described. Upon treatment of the lesion using the proton therapy system 1, the treatment plan using a CT image obtained by imaging the patient is created in advance, and a proton beam from the proton therapy system 1 is emitted to the lesion on the basis of the treatment plan. However, since a CT image used when creating the treatment plan is formed using a different apparatus from the proton therapy system 1, the position of the patient when capturing a CT image for treatment planning does not necessarily match the position of the patient upon treatment using the proton therapy system 1. In this case, if the patient cannot be placed at the correct position on the treatment table 11 of the proton therapy system 1, the position of the lesion of the patient in the proton therapy system 1 becomes inaccurate. As a result, a proton beam may not be emitted to the correct position of the lesion unlike the treatment plan. On the other hand, in order to minimize errors due to misalignment of the position of the patient, the CT image creation apparatus for charged particle beam therapy according to the present embodiment creates a cone beam CT image (CT image for particle beam therapy) on the proton therapy system 1, which is an apparatus used for treatment, using the X-ray tubes 31 and the FPDs 32 mounted in the proton therapy system 1 and then compares the created cone beam CT image with a CT image for treatment planning created in advance and corrects the position of the patient in the proton therapy system 1.

In addition, in the present embodiment, a case will be described in which a CT image is acquired by capturing an X-ray image using a phantom in which a component through which X-rays are not transmitted is provided and the size of the main body and the position and size of the internal component are evident, data for position calibration is first created by performing correction to remove the roughness of the CT image, and then the CT image is reconstructed by correcting X-ray image data obtained by imaging the patient using the data for position calibration. Instead of the above-described method of creating data for position calibration using a phantom and reconstructing a corrected CT image using the data for position calibration, it is also possible to adopt a method of correcting each CT image, which is obtained by imaging the patient, without using the data for position calibration obtained by imaging a phantom. In this method, however, the correction is performed without using a phantom whose exact position is known. Accordingly, an improvement in the accuracy of the obtained CT image in the case of correction using a phantom is much higher than that in the case of correction using no phantom.

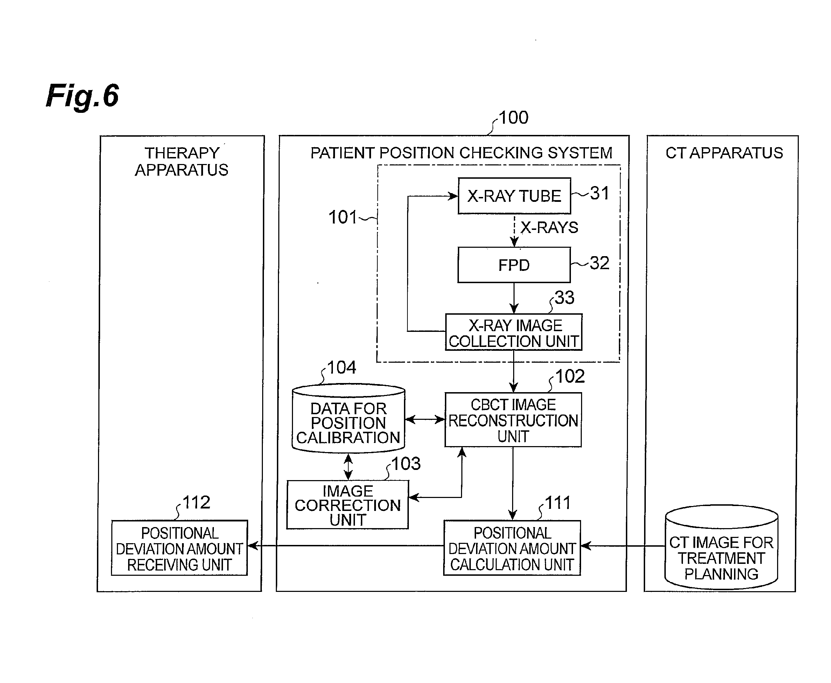

Fig. 6 is a diagram illustrating the configuration of a CT image creation apparatus for charged particle beam therapy.

A CT image creation apparatus for charged particle beam therapy 100 according to the present embodiment (patient position checking system) is configured to include an X-ray image acquisition unit 101 (image acquisition unit), an image reconstruction unit 102 (reconstruction unit), an image correction unit 103 (first to third detection units and first to third correction units), a DB for position calibration 104, and a positional deviation amount calculation unit 111. In addition, a positional deviation amount receiving unit 112 that receives a result calculated by the positional deviation amount calculation unit 111 is provided in a proton therapy unit. Among these, the X-ray image acquisition unit 101, the image reconstruction unit 102, and the image correction unit 103 are used to create data (data for position calibration) for performing position calibration and create a CT image of the patient placed on the treatment table 11. In addition, the positional deviation amount calculation unit 111 has a function of correcting a CT image, which is obtained by imaging the patient in the proton therapy system 1, using the data for position calibration stored in the DB for position calibration 104 and then calculating the amount of positional deviation from the CT image for treatment planning captured in advance to make a treatment plan. In addition, Fig. 6 shows a case where the CT image for treatment planning is information provided by performing imaging using a different CT apparatus from the system 1 and the positional deviation amount information is used in proton therapy.

The X-ray image acquisition unit 101 is configured to include the X-ray tube 31, the FPD 32, and an X-ray image collection unit 33. The X-ray image acquisition unit 101 has a function of acquiring an X-ray image. The X-ray image used to generate the data for position calibration is an image with an internal configuration that is obtained by imaging a known phantom. An X-ray image captured by the FPD 32 using X-rays emitted from the X-ray tube 31 and transmitted through a phantom is collected so as to match the imaging time in the X-ray image collection unit 33. Here, a phantom is imaged from a plurality of directions by capturing a plurality of X-ray images every predetermined angle set in advance while rotating the rotating gantry 7. By collecting the imaging time and the X-ray image so as to match each other in the X-ray image collection unit 33, the position and the X-ray image of the rotating gantry 7 are collected so as to match each other.

The image reconstruction unit 102 has a function of reconstructing a CT image from an X-ray image acquired by the X-ray image acquisition unit 101. In addition, the image reconstruction unit 102 has a function of reconstructing a CT image even if an X-ray image corrected by the image correction unit 103 at the subsequent stage is used. Images reconstructed by the image reconstruction unit 102 include an X-ray image of a phantom and an X-ray image of a patient.

The image correction unit 103 has a function of determining whether or not position calibration, which will be described later, is necessary for an X-ray image, which is used in order to reconstruct the CT image, on the basis of the CT image reconstructed by the image reconstruction unit 102 and correcting the X-ray image on the basis of the determination result.

The DB for position calibration 104 is a database that stores information related to the positional deviation in the apparatus to capture an X-ray image, that is, information for performing position calibration on the basis of an X-ray image obtained by imaging a phantom and a CT image reconstructed from the X-ray image. The information related to the positional deviation of the apparatus obtained by repeating the CT image reconstruction in the image reconstruction unit 102 and the X-ray image correction in the image correction unit 103 is stored in the DB for position calibration 104.

When the CT image obtained by imaging the patient is modified using the information stored in the DB for position calibration 104, the X-ray image collection unit 33 collects X-ray image data, which is obtained by imaging using the X-ray tube 31 and the FPD 32 of the proton therapy system 1 according to the present embodiment, and the image reconstruction unit 102 reconstructs the CT image. Then, the image correction unit 103 corrects the X-ray image data, which forms the CT image, using the data for position calibration stored in the DB for position calibration 104. Then, the image reconstruction unit 102 reconstructs the CT image based on the X-ray image data after correction and outputs the reconstructed CT image to the image display device or the like. In this manner, the CT image after correction can be used as a CT image for particle beam therapy.

The positional deviation amount calculation unit 111 calculates the amount of positional deviation between the CT image of the patient obtained by the image reconstruction unit 102 and the CT image for treatment planning captured in advance to make a treatment plan. Then, this information is transmitted to the positional deviation amount receiving unit 112. In addition, in the proton therapy unit, after receiving the information in the positional deviation amount receiving unit 112, for example, the proton beam emission position is changed or the treatment table 11 is moved to change the position of the patient on the basis of the positional deviation information in order to emit a proton beam appropriately to the lesion of the patient on the basis of the treatment plan.

Here, the positional deviation of the apparatus calculated when creating the data for position calibration may become error of the apparatus in proton therapy and affect proton therapy. Accordingly, by adopting the configuration in which the positional deviation amount calculation unit 111 calculates the amount of positional deviation on the basis of data for position calibration and the data for position calibration is transmitted to the proton therapy unit, such information can also be used when controlling the rotation of the rotating gantry 7 at the time of proton therapy or adjusting the radiation position of a proton beam.

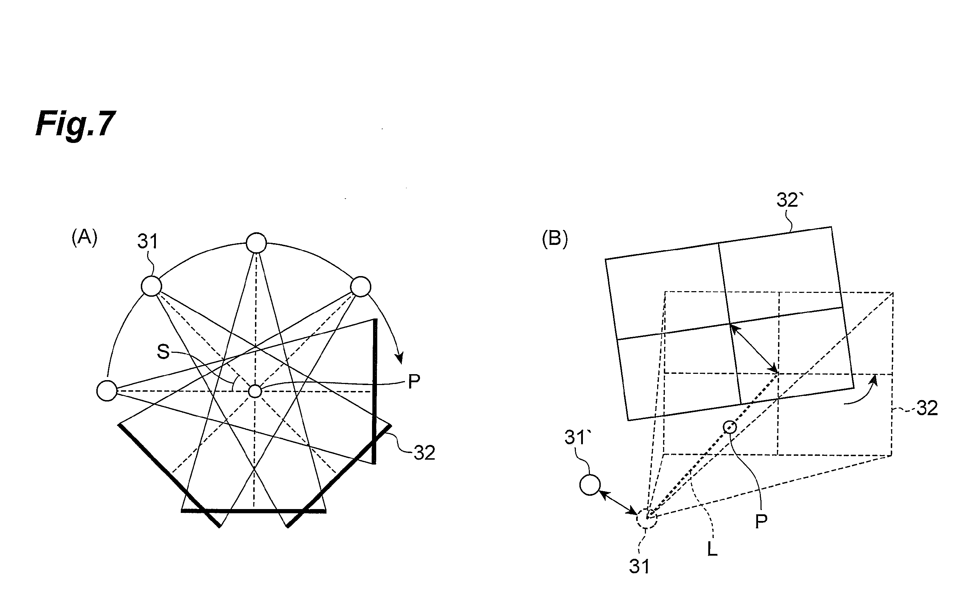

Here, positional deviation occurring in the apparatus when the X-ray image data of an object to be measured is imaged using the X-ray tube 31 and the FPD 32 fixed to the rotating gantry 7 will be described with reference to Figs. 7A and 7B.

Since a particle beam therapy apparatus such as the proton therapy system 1 is large and heavy, a CT image (CT image for charged particle beam therapy) obtained by the CT imaging apparatus fixed to the charged particle beam therapy apparatus for the purpose of positioning of the patient is not clear. This is because the actual imaging position of X-ray image data is different from the position set in advance, that is, an image captured in a state where positional deviation has occurred is included when reconstructing a CT image after imaging the X-ray image data of a plurality of objects to be measured. In addition, if a CT image acquired for the purpose of positioning of the patient at the time of proton therapy in this proton therapy system 1 is not clear, comparison with a CT image used when creating a treatment plan is difficult. As a result, the positioning of the patient at the time of proton therapy becomes difficult. For this reason, there is a problem in that a proton beam cannot be correctly emitted to the lesion of the patient on the basis of a treatment plan.

The following three causes may be mentioned as the main causes of "positional deviation" that makes unclear a CT image reconstructed on the basis of the X-ray image data imaged in the proton therapy system 1.

(1) The rotation angle of the rotating gantry 7 deviates from the predetermined value
(2) The position of the X-ray tube 31 deviates from the set position
(3) The position of the FPD 32 (X-ray detector) deviates from the set position

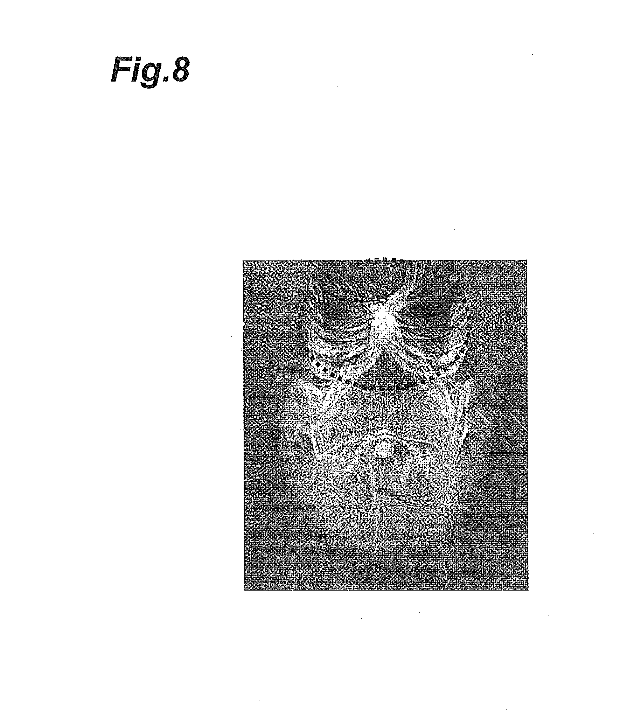

Among these causes, for (1) rotation angle, for example, as shown in Fig. 7(A), when the rotation angle of the rotating gantry 7 is $S \pm \alpha$ although the rotating gantry 7 is originally to be rotated by an angle S with respect to the shaft center P to perform imaging, there is a problem in that the imaging position deviates from the position assumed initially. If an image is reconstructed without modifying the error when such angle deviation occurs, a CT image after reconstruction is not clear. Fig. 8 shows an example when reconstructing an image without correcting X-ray image data obtained by imaging a phantom. In Fig. 8, arc-shaped artifacts (region looking white in an arc shape) are identified in a region indicated by the dotted line. Thus, the rotation angle deviation of the rotating gantry 7 can be identified as arc-shaped artifacts in the X-ray image data.

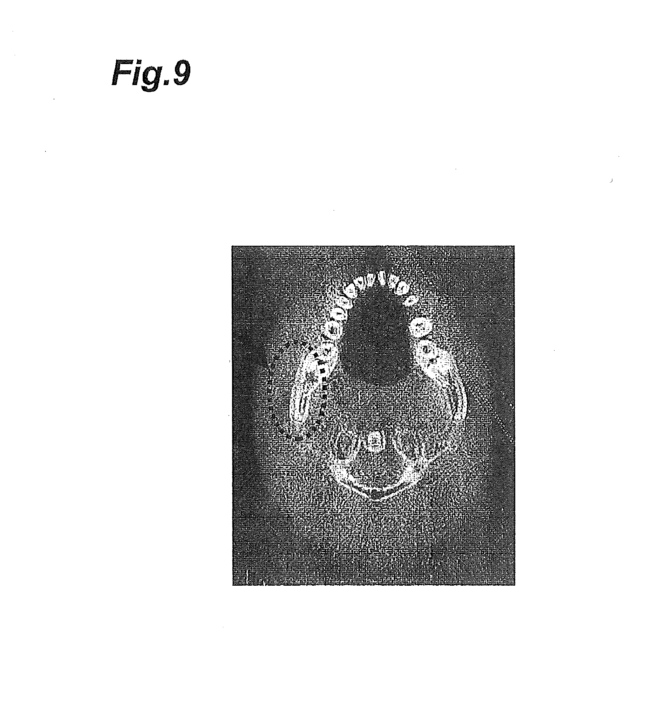

In addition, for (2) positional deviation of the X-ray tube and (3) positional deviation of the FPD, for example, as shown in Fig. 7(B), when the position deviates like an X-ray tube 31' or an FPD 32' although the shaft center P and the emission port of the X-ray tube 31 are to be provided on the perpendicular to the detector surface of the FPD 32 extending from the center of the FPD 32, a CT image after reconstruction is not clear if an image is reconstructed without modifying the error. Fig. 9 shows an example when reconstructing an image without correcting X-ray image data obtained by imaging a phantom. In Fig. 9, a region where an image is blurred is generated in a region indicated by the dotted line. Thus, positional deviation of the X-ray tube and the FPD can be identified as a blurred image in X-ray image data.

Among the deviations due to the apparatus in the above (1) to (3), the influence of (1) angle deviation of the rotating gantry is particularly larger than the influence of the other (2) and (3).

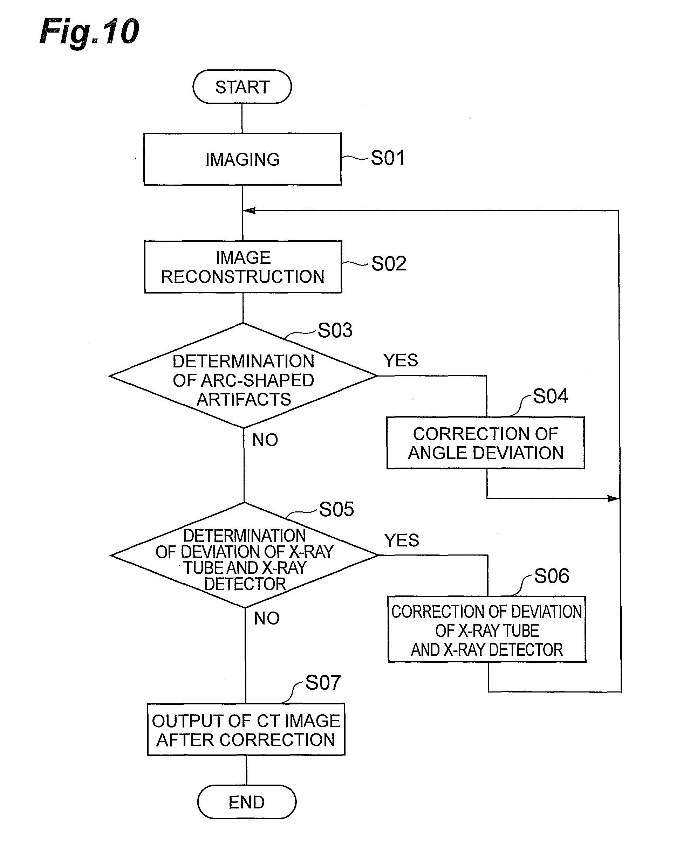

Therefore, for image correction for creating the CT image for charged particle beam therapy according to the present embodiment, correction is performed for (1) angle deviation of the rotating gantry and then correction is performed for (2) positional deviation of the X-ray tube and (3) positional deviation of the FPD. The specific method will be described using the flow chart shown in Fig. 10.

First, as shown in Fig. 10, an image is captured by the X-ray tube 31 and the FPD 32 included in the X-ray image acquisition unit 101 while rotating the rotating gantry 7, and the X-ray image data is collected by the X-ray image collection unit 33 (S01). Then, the image reconstruction unit 102 reconstructs a CT image from the X-ray image data of a plurality of images (S02). Then, the image correction unit 103 determines whether or not arc-shaped artifacts can be identified in the reconstructed CT image (S03).

Here, when arc-shaped artifacts cannot be identified, the process proceeds to the next step. However, when arc-shaped artifacts can be identified, angle deviation correction is performed (S04). For the artifact correction, deviation ($\pm \alpha$ when the rotation angle is $S \pm \alpha$) of the angle of the rotating gantry is calculated on the basis of the size, shape, and strength of the artifacts and correction for removing this angle deviation is performed (S05). After correcting the angle deviation, the image reconstruction unit 102 reconstructs the X-ray image data (S02), and checks whether or not arc-shaped artifacts appear in the obtained CT image (S03). Components of the angle deviation of the rotating gantry are removed by repeating the above until the arc-shaped artifacts disappear in the CT image after reconstruction.

Then, in the CT image from which arc-shaped artifacts have been removed, the presence of positional deviation of the X-ray tube 31 and the FPD 32 is checked on the basis of whether or not there is roughness of an image due to positional deviation of the X-ray tube 31 and the FPD 32 (detector) (S05). Here, when the roughness of the image due to the X-ray tube and the FPD is identified, the X-ray image data is corrected to remove the roughness (S06). Then, on the basis of the X-ray image data after correction, the image is reconstructed by the image reconstruction unit 102 (S02), it is checked whether or not there are arc-shaped artifacts (S03), and it is checked whether or not there is deviation due to the X-ray tube and the detector (S05). When it is confirmed that all of these have been removed, the CT image after correction is output to the image display device or the like and the parameters used in rotation angle deviation correction, X-ray tube deviation correction, and detector deviation correction are stored in the DB for position calibration 104 as data for position calibration.

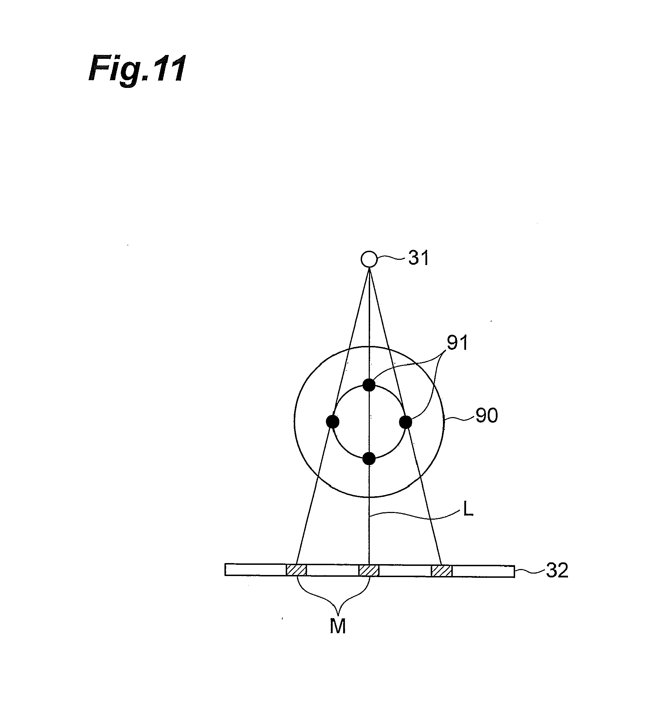

Here, how to distinguish the deviation of the X-ray tube from the deviation of the detector in order to perform correction will be described with reference to Figs. 11 to 15.

Fig. 11 is a diagram illustrating a case (ideal state) where the X-ray tube 31 and the FPD 32 are disposed at the normal positions, that is, the X-ray tube 31 is provided on the perpendicular L that extends from the center of the light receiving surface on the light receiving surface of the FPD 32. In Fig. 11, a phantom 90 serving as an object to be measured is disposed between the X-ray tube 31 and the FPD 32. Four metals 91 displayed in white in the X-ray image data are provided inside the phantom 90, and two of them are assumed to be on the perpendicular L. Here, when the X-ray tube 31 and the FPD 32 are disposed at the normal positions, projection in a state where the two metals 91 on the perpendicular L overlap each other is realized. Accordingly, in the X-ray image data imaged by the FPD 32, a white region M corresponding to the metal 91 appears in three places.

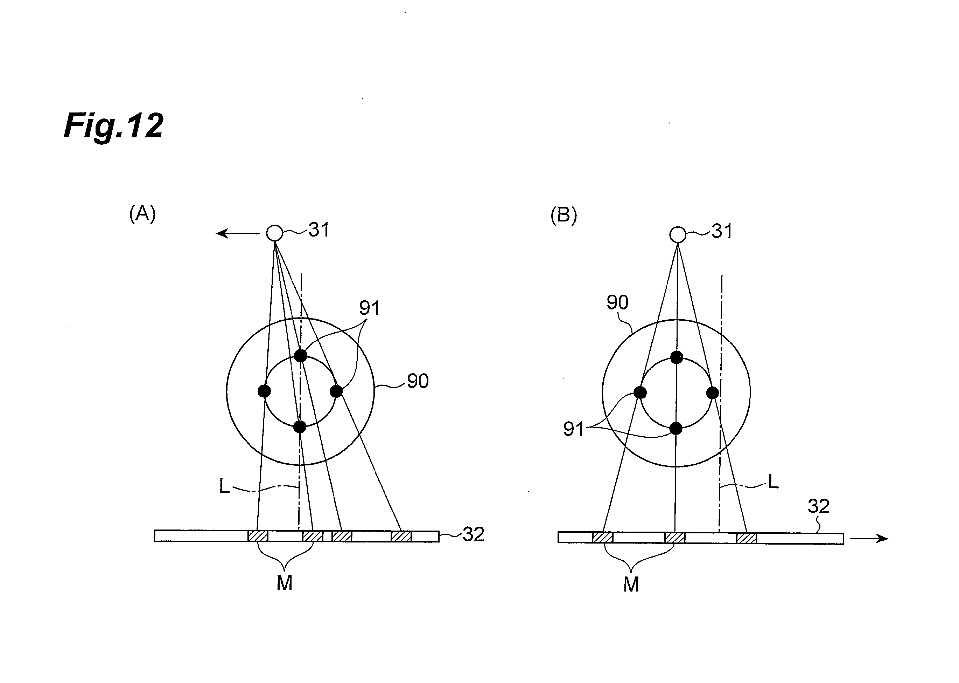

On the other hand, when the X-ray tube 31 deviates in the arrow direction (leftward in Fig. 12(A)) from the original position as shown in Fig. 12(A), projection in a state where the two metals 91 on the perpendicular L overlap each other is not realized. Accordingly, in the X-ray image data imaged by the FPD 32, a white region M corresponding to the metal 91 appears in four places. Thus, when the position of the X-ray tube 31 with respect to the FPD 32 deviates, the angle of the light receiving surface of the FPD 32 with respect to cone beams of X-rays emitted from the X-ray tube 31 changes as a result. In addition, as shown in Fig. 12 (B), when the FPD 32 deviates in parallel with the arrow direction (rightward in Fig. 12(B)) from the original position (that is, when there is no positional deviation in a vertical direction), there is no change in the positional relationship between the X-ray tube 31 and the phantom 90 compared with a normal case. Accordingly, in the X-ray image data imaged by the FPD 32, three white regions M corresponding to the metal 91 appear, but the positions where the white regions M are formed are different from the original positions of the FPD 32.

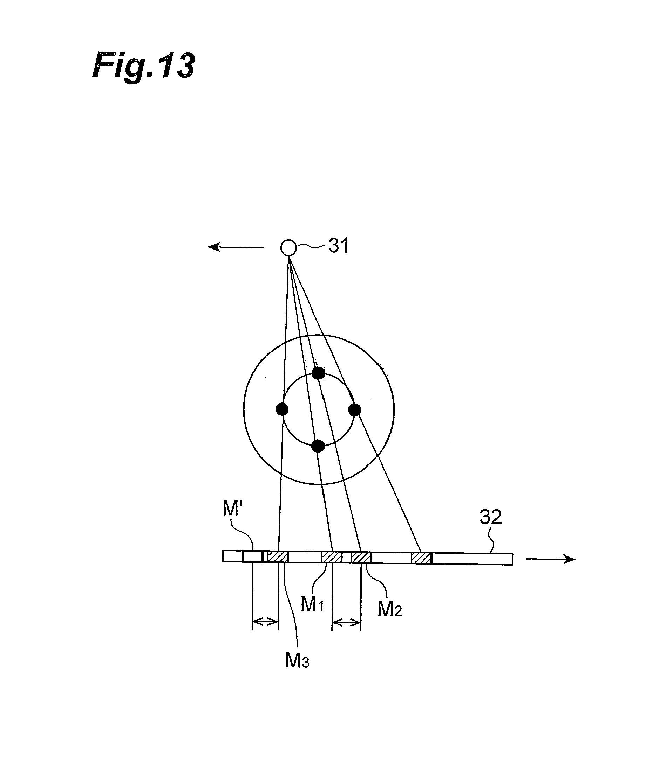

Thus, the image deviation occurring due to the positional deviation of the X-ray tube 31 is different from the positional deviation of the FPD 32. Accordingly, on the basis of the above, when both the positions of the X-ray tube 31 and the FPD 32 deviate, the amount of positional deviation is first calculated using the following method. Here, as shown in Fig. 13, it is assumed that the position of the X-ray tube 31 deviates in the left direction in the drawing and the position of the FPD 32 deviates in the right direction in the drawing. In this case, in the X-ray image data obtained as a result of imaging the same phantom 90 as in Figs. 11, 12A, and 12B, a white region M corresponding to the metal 12 appears at four places. Among them, the middle regions $M_1$ and $M_2$ are white regions that should overlap each other if the X-ray tube 31 is in an ideal state. In addition, a region M' surrounded by the dotted line shown in Fig. 13 shows a region considered that a white region appears in the case of imaging in the ideal state, but the region $M_3$ appears due to the positional deviation of the X-ray tube 31 and the FPD 32. Therefore, the amount of positional deviation of the FPD 32 can be calculated by calculating the amount of positional deviation of the X-ray tube 31 from the ideal position on the basis of the distance between the regions $M_1$ and $M_2$ first and then removing components due to the positional deviation of the X-ray tube 31 from the distance between the regions M' and $M_3$. Thus, by distinguishing the amount of positional deviation of the X-ray tube 31 and the amount of positional deviation of the FPD 32 from each other on the basis of the X-ray image data obtained by imaging the phantom 90, it is possible to calculate the amount of positional deviation of the X-ray tube 31 and the amount of positional deviation of the FPD 32.

In the above, the positional deviation of the FPD 32 has been described above on the assumption that there is no change in the angle of the light receiving surface. However, when positional deviation is caused to change the angle of the light receiving surface, it is thought that the same event as in the case of the positional deviation of the X-ray tube 31 occurs. When it is necessary to calculate the amount of positional deviation of the X-ray tube 31 and the amount of positional deviation of the FPD 32 accurately, the influence in the case of the positional deviation of the X-ray tube 31 and the influence in the case of the positional deviation of the FPD 32 need to be more specifically examined and reflected. In the cases of the positional deviation of the X-ray tube 31 and the positional deviation of the FPD 32, however, the influence of the image roughness in the CT image after reconstruction is small compared with a case of rotation angle deviation of the rotating gantry 7. Therefore, when it is not necessary to calculate the amount of positional deviation accurately, image correction can be performed using the following method.

Next, a method of performing correction after calculating the amount of positional deviation of the X-ray tube 31 and the FPD 32 will be described. Here, (1) correction of components due to positional deviation in a state where the X-ray tube and the FPD are parallel and (2) correction of components due to angle deviation of the FPD with respect to the X-ray tube will be described. (1) may occur when the FPD moves in parallel from the ideal state. In addition, (2) may occur when the position of the X-ray tube deviates from the ideal state and when the FPD moves in a vertical direction.

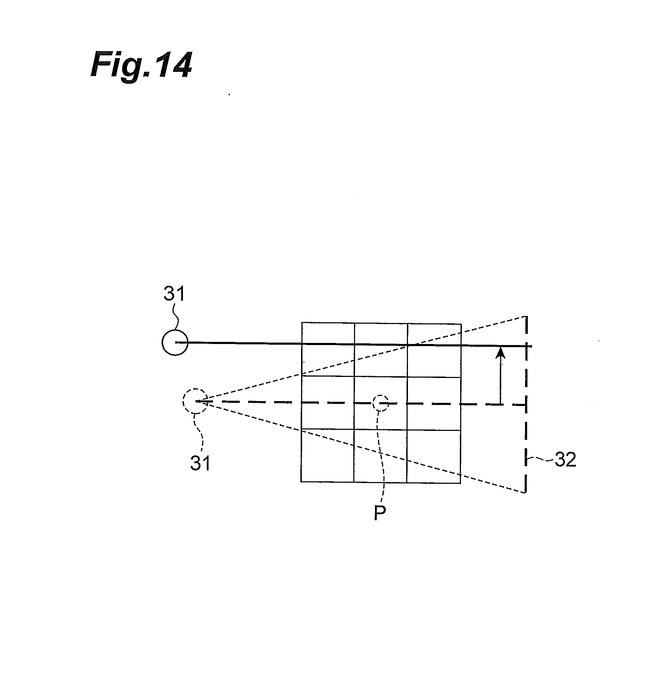

First, (1) correction of components due to positional deviation in a state where the X-ray tube and the FPD are parallel will be described. In this case, as shown in Fig. 14, since the intensity of X-rays received in each pixel is different from that in the ideal state, X-ray image data obtained as a result is different from that in the ideal state, but is in a state of positional deviation on the whole compared with image data to be imaged in the ideal state. Accordingly, it is possible to obtain the X-ray image data in the ideal state by shifting the information of each pixel on the basis of the amount of positional deviation, that is, by shifting the X-ray intensity of each pixel by the amount of positional deviation in all pixels.

Next, (2) correction of components due to angle deviation of the FPD with respect to the X-ray tube will be described.

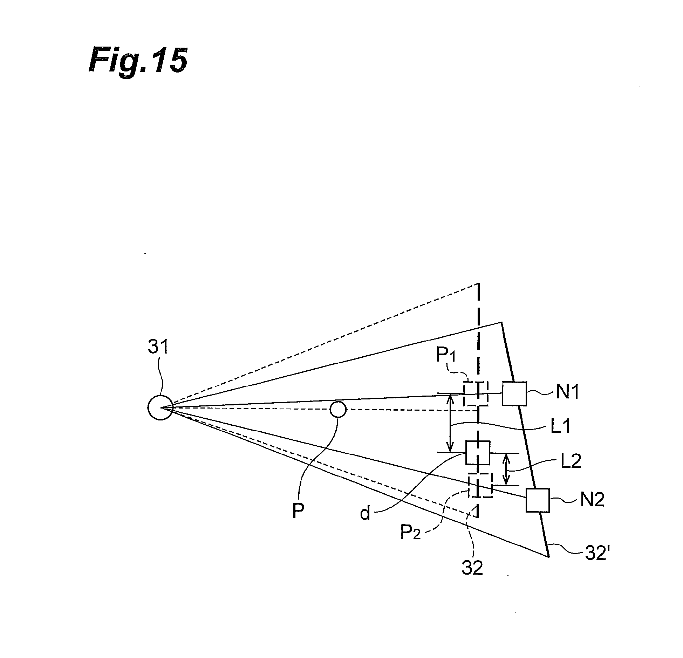

In the case of (1), for the intensity of X-rays received in each pixel, the light receiving positions in the ideal state and the positional deviation state are different but the same information is received. In the case of (2), however, the intensity of X-rays received in each pixel is different from that in the ideal state. Therefore, data is created using a method of linear interpolation. In Fig. 15, a method of acquiring data D at the position d on the FPD 32 in the ideal state on the basis of data items N1 and N2 that are acquired at two shifted positions obtained in an FPD 32' disposed at a different position from that in the ideal state will be described. The data items N1 and N2 are X-rays transmitted through P1 and P2 on the FPD 32, respectively. Here, assuming that the distance between P1 and d is L1 and the distance between P2 and d is L2, the data D at the position d in the FPD 32 in the ideal state can be calculated using the following Expression (1).

$$D = N1 \times L2/(L1 + L2) + N2 \times L1/(L1 + L2) \cdots (1)$$

By calculating this for each pixel on the FPD 32 in the ideal state, it is possible to calculate the data in the FPD 32 in the ideal state.

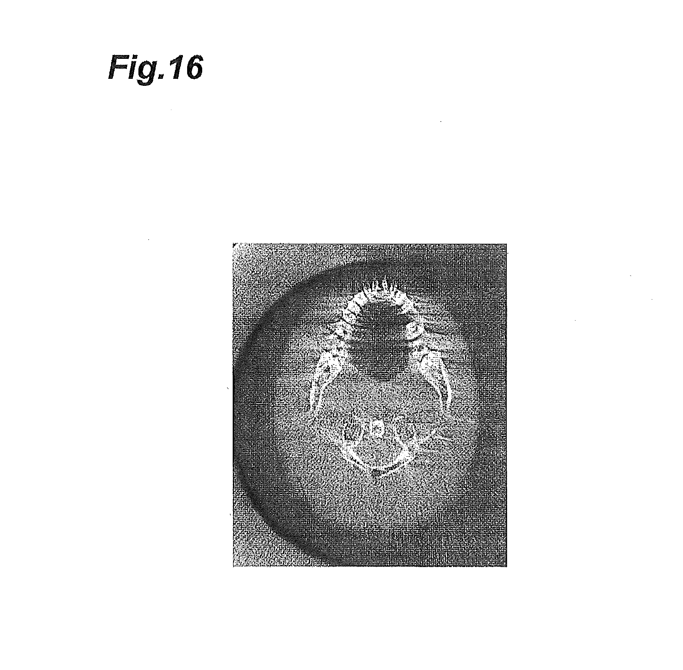

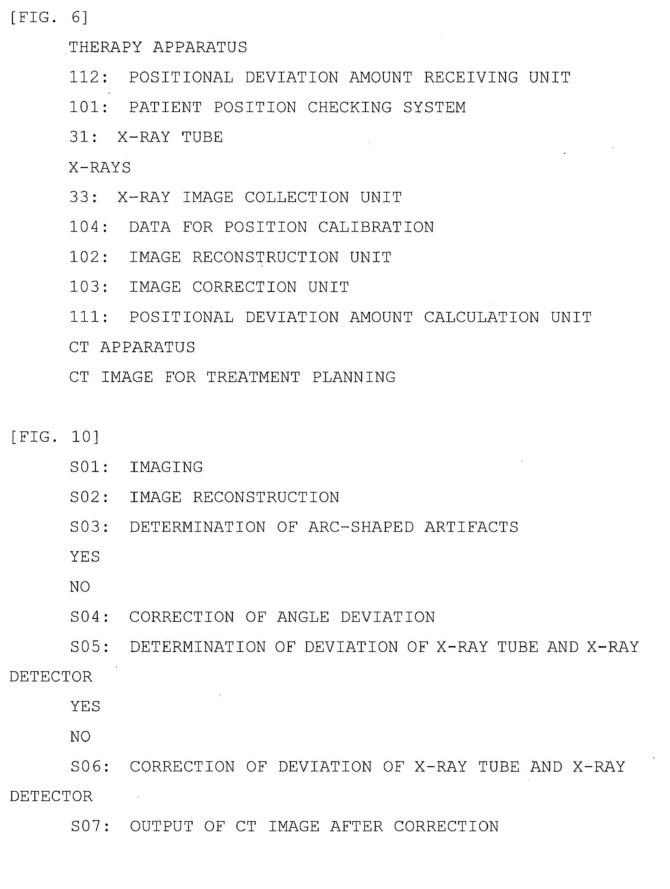

Finally, the result of the above-described correction is shown in Fig. 16. Fig. 16 is a CT image reconstructed after performing correction relevant to the removal of arc-shaped artifacts, that is, correction of the rotation angle of the rotating gantry for the phantom image shown in Fig. 8 and performing correction relevant to the removal of image roughness due to positional deviation of the X-ray tube and the detector for the phantom image shown in Fig. 9. When Fig. 16 is compared with Fig. 8, arc-shaped artifacts are removed. In addition, when Fig. 16 is compared with Fig. 9, arc-shaped artifacts are removed. In the image of the dotted line portion shown in Fig. 9, two white regions shifted from each other are seen. However, a clear white region is seen in Fig. 16. Thus, by performing the correction shown above, image roughness in a CT image can be corrected. As a result, a CT image for charged particle beam therapy can be created more accurately.

As described above, according to the proton therapy system 1 including the CT image creation apparatus for charged particle beam therapy according to the present embodiment, a CT image for charged particle beam therapy is created by detecting the deviation of the angle of the rotating gantry 7 from the predetermined angle when capturing X-ray image data on the basis of a CT image and correcting the X-ray image data on the basis of the detected deviation to reconstruct the CT image. For this reason, even in the large and heavy apparatus such as the proton therapy system 1, it is possible to modify the distortion of CT images due to the deviation of the angle of the rotating gantry 7 in a CT image for charged particle beam therapy obtained for the purpose of positioning of the patient. As a result, it is possible to create a more accurate CT image for charged particle beam therapy. In addition, since the positioning of the patient at the time of proton beam irradiation in the proton beam irradiation system 1 can be performed more accurately on the basis of the high-accuracy CT image for charged particle beam therapy, a proton beam can be accurately emitted to the lesion of the patient on the basis of a treatment plan.

In addition, by adopting the configuration in which the deviation of the fixing position of the X-ray tube 31 is detected on the basis of a CT image and the X-ray image data is corrected on the basis of the detected deviation to reconstruct the CT image as in the embodiment described above, a more accurate CT image can be created.

In addition, by adopting the configuration in which the deviation of the fixing position of the FPD 32, which is an X-ray detector, is detected on the basis of a CT image and the X-ray image data is corrected on the basis of the detected deviation to reconstruct the CT image, a more accurate CT image can be created.

Inaddition, in the reconstructed CT image, imagedistortion due to the deviation of the angle of the rotating gantry 7, among various kinds of positional deviation of the apparatus related to the capturing of a CT image, is largest. Accordingly, the accuracy of correction of a CT image is further improved by performing a modification based on the deviation of the fixing position of the X-ray tube 31 and the deviation of the fixing position of the FPD 32 after performing correction related to the deviation of the angle of the rotating gantry 7.

While the present invention has been specifically described on the basis of the embodiment, the present invention is not limited to the above embodiment. For example, although the configuration in which the CT image creation apparatus for charged particle beam therapy is included in the proton therapy system has been described in the above embodiment, the CT image creation apparatus for charged particle beam therapy according to the present embodiment may be provided separately from the proton therapy system. That is, the CT image creation apparatus for charged particle beam therapy according to the present embodiment is an apparatus that corrects and outputs a CT image, which is captured in the proton therapy system in which the X-ray tube and the X-ray detector are fixed to the rotating gantry including a mechanism for irradiating a proton beam, so as to be able to be used as a CT image for charged particle beam therapy. Therefore, a configuration as a CT image creation apparatus that acquires a CT image, which is captured in the proton therapy system in which the X-ray tube and the X-ray detector are fixed to the rotating gantry including a mechanism for irradiating a proton beam, from the outside and corrects the acquired CT image can be provided separately from the proton therapy system.

It should be understood that the invention is not limited to the above-described embodiment, but may be modified into various forms on the basis of the spirit of the invention. Additionally, the modifications are included in the scope of the invention.

**Claims**

1. A CT image creation apparatus for charged particle beam therapy, comprising:

    an image acquisition unit that acquires X-ray image data, which is imaged every predetermined set rotation angle, while rotating a rotating gantry to which an X-ray tube that emits X-rays and an X-ray detector that detects X-rays emitted from the X-ray tube are fixed;
    a reconstruction unit that reconstructs a CT image on the basis of the X-ray image data;
    a first detection unit that detects a difference between an angle of the rotating gantry, at which the X-ray image data has been imaged, and the predetermined rotation angle on the basis of the CT image; and
    a first correction unit that corrects the X-ray image data on the basis of a detection result of the first detection unit, wherein the reconstruction unit reconstructs the CT image on the basis of X-ray image data corrected by the first correction unit.

2. The CT image creation apparatus for charged particle beam therapy according to claim 1, further comprising:

    a second detection unit that detects a difference between a set fixing position of the X-ray tube and a fixing position of the X-ray tube, at which the X-ray image data has been imaged, on the basis of the CT image; and
    a second correction unit that corrects the X-ray image data on the basis of a detection result of the second detection unit,
    wherein the reconstruction unit reconstructs the CT image on the basis of X-ray image data corrected by the second correction unit.

3. The CT image creation apparatus for charged particle beam therapy according to claim 1, further comprising:

a third detection unit that detects a difference between a set fixing position of the X-ray detector and a fixing position of the X-ray detector in the rotating gantry, at which the X-ray image data has been imaged, on the basis of the CT image; and
a third correction unit that corrects the X-ray image data on the basis of a detection result of the third detection unit, wherein the reconstruction unit reconstructs the CT image on the basis of X-ray image data corrected by the third correction unit.

4. The CT image creation apparatus for charged particle beam therapy according to claim 2, wherein the second detection unit detects deviation of the fixing position of the X-ray tube in the rotating gantry, at which the X-ray image data has been imaged, on the basis of the X-ray image data corrected by the first correction unit.

5. The CT image creation apparatus for charged particle beam therapy according to claim 3, wherein the third detection unit detects deviation of the fixing position of the X-ray tube in the rotating gantry, at which the X-ray image data has been imaged, on the basis of the X-ray image data corrected by the first correction unit.

Fig.1

**Fig.2**

**Fig.3**

EP 2 687 261 A1

## Fig.4

Fig.5

**Fig.6**

CT APPARATUS

CT IMAGE FOR TREATMENT PLANNING

PATIENT POSITION CHECKING SYSTEM — 100

101

X-RAY TUBE — 31

X-RAYS

FPD — 32

33 — X-RAY IMAGE COLLECTION UNIT

102 — CBCT IMAGE RECONSTRUCTION UNIT

104 — DATA FOR POSITION CALIBRATION

103 — IMAGE CORRECTION UNIT

111 — POSITIONAL DEVIATION AMOUNT CALCULATION UNIT

THERAPY APPARATUS

112 — POSITIONAL DEVIATION AMOUNT RECEIVING UNIT

# *Fig.7*

(A)

(B)

EP 2 687 261 A1

Fig.8

Fig.9

# Fig.10

```
          ┌─────────┐
          │  START  │
          └────┬────┘
               │
    ┌──────────┴──────────┐
    │       IMAGING       │──── S01
    └──────────┬──────────┘
               │◄──────────────────────────────────────┐
               │                                        │
    ┌──────────┴──────────┐                             │
    │        IMAGE        │──── S02                      │
    │    RECONSTRUCTION   │                              │
    └──────────┬──────────┘                             │
               │        S03                             │
            ╱──┴──╲                                      │
          ╱ DETERMINATION ╲      YES                     │
        ╱  OF ARC-SHAPED   ╲──────────┐                  │
        ╲    ARTIFACTS     ╱          │   S04            │
          ╲──┬──╱              ┌──────┴───────────┐      │
             │ NO              │  CORRECTION OF   │      │
             │                 │ ANGLE DEVIATION  │      │
             │                 └──────────────────┘──────┤
             │        S05                                │
          ╱──┴──╲                                        │
        ╱  DETERMINATION ╲      YES                       │
      ╱ OF DEVIATION OF X-RAY ╲──────────┐                │
      ╲   TUBE AND X-RAY     ╱           │    S06          │
      ╲    DETECTOR         ╱     ┌───────┴──────────────┐ │
          ╲──┬──╱                │CORRECTION OF DEVIATION│ │
             │ NO                │    OF X-RAY TUBE      │ │
             │                   │  AND X-RAY DETECTOR   │ │
             │                   └──────────────────────┘─┘
             │        S07
    ┌────────┴──────────┐
    │ OUTPUT OF CT IMAGE│
    │  AFTER CORRECTION │
    └────────┬──────────┘
             │
        ┌────┴────┐
        │   END   │
        └─────────┘
```

# Fig.11

Fig.12

# Fig.13

# Fig.14

# Fig.15

Fig.16

[FIG. 6]

    THERAPY APPARATUS

    112: POSITIONAL DEVIATION AMOUNT RECEIVING UNIT

    101: PATIENT POSITION CHECKING SYSTEM

    31: X-RAY TUBE

    X-RAYS

    33: X-RAY IMAGE COLLECTION UNIT

    104: DATA FOR POSITION CALIBRATION

    102: IMAGE RECONSTRUCTION UNIT

    103: IMAGE CORRECTION UNIT

    111: POSITIONAL DEVIATION AMOUNT CALCULATION UNIT

    CT APPARATUS

    CT IMAGE FOR TREATMENT PLANNING


[FIG. 10]

    S01: IMAGING

    S02: IMAGE RECONSTRUCTION

    S03: DETERMINATION OF ARC-SHAPED ARTIFACTS

    YES

    NO

    S04: CORRECTION OF ANGLE DEVIATION

    S05: DETERMINATION OF DEVIATION OF X-RAY TUBE AND X-RAY DETECTOR

    YES

    NO

    S06: CORRECTION OF DEVIATION OF X-RAY TUBE AND X-RAY DETECTOR

    S07: OUTPUT OF CT IMAGE AFTER CORRECTION

EP 2 687 261 A1

Europäisches
Patentamt
European
Patent Office
Office européen
des brevets

# EUROPEAN SEARCH REPORT

Application Number

EP 13 02 0052

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BORIS P. SELBY ET AL: "Geometry calibration for x-ray equipment in radiation treatment devices and estimation of remaining patient alignment errors", PROCEEDINGS OF SPIE, vol. 6913, 6 March 2008 (2008-03-06), page 691355, XP055087110, ISSN: 0277-786X, DOI: 10.1117/12.770294 * page 1 - page 8; figures 1,2,3,4,5 * | 1-5 | INV. A61N5/10 G06T11/00 |
| A | BORIS PETER SELBY ET AL: "Patient positioning with X-ray detector self-calibration for image guided therapy", AUSTRALASIAN PHYSICAL & ENGINEERING SCIENCES IN MEDICINE, vol. 34, no. 3, 2 August 2011 (2011-08-02) , pages 391-400, XP055087112, ISSN: 0158-9938, DOI: 10.1007/s13246-011-0090-4 * page 393, right-hand column - page 395; table 1 * * figures 1,2 * | 1-5 | |
| A | ALFRED R SMITH: "REVIEW; Proton therapy", PHYSICS IN MEDICINE AND BIOLOGY, INSTITUTE OF PHYSICS PUBLISHING, BRISTOL GB, vol. 51, no. 13, 7 July 2006 (2006-07-07), pages R491-R504, XP020095840, ISSN: 0031-9155, DOI: 10.1088/0031-9155/51/13/R26 * the whole document * | 1-5 | TECHNICAL FIELDS SEARCHED (IPC) A61N G06T |

The present search report has been drawn up for all claims

| Place of search | Date of completion of the search | Examiner |
|---|---|---|
| Munich | 7 November 2013 | Werling, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 2 687 261 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012161796 A